# FASCICULE DE BREVET EUROPEEN

(11) **EP 0 830 450 B1**
(45) Date de publication et mention de la délivrance du brevet: **11.01.2006**
(21) Numéro de dépôt: 96914243.9
(22) Date de dépôt: 25.04.1996
(51) Int. Cl.: C12N 15/12, C12N 15/55, C12N 5/10, C07K 16/18, G01N 33/50, A61K 35/12, A61K 39/395

(54) **LIGNEES IMMORTALISEES DE CELLULES ISSUES DU TISSU ADIPEUX HUMAIN, LEUR PROCEDE DE PREPARATION ET LEURS APPLICATIONS**
IMMORTALIZIERTE LINIEN VON AUS MENSCHLICHEN FETTGEWEBE ABSTAMMENDEN ZELLEN, VERFAHREN ZU IHRER HERSTELLUNG UND IHRER VERWENDUNGEN
IMMORTALISED CELL LINES FROM HUMAN ADIPOSE TISSUE, PROCESS FOR PREPARING SAME AND APPLICATIONS THEREOF

(30) Priorité: 25.04.1995 FR 9504922
(43) Date de publication de la demande: 25.03.1998
(73) Titulaire: CENTRE NATIONAL DE LA RECHERCHE SCIENTIFIQUE (CNRS), 75794 Paris Cedex 16 (FR)
(72) Inventeur: STROSBERG, Arthur, Donny, F-75015 Paris (FR); ZILBERFARB, Vladimir, F-91140 Villebon-sur-Yvette (FR)
(74) Mandataire: Orès, Irène
(86) Numéro de dépôt international: PCT/FR1996/000634
(87) Numéro de publication internationale: WO 1996/034100

(56) Documents cités:
- EP-A- 0 409 696
- US-A- 5 102 658
- MOL.CELL.BIOL, vol. 4, no. 4, pages 712-721, XP002013115 YASUMOTO: "HORMONAL REGULATION OF THE TRANSFORMATION PHENOTYPE IN SIMIAN VIRUS 40-TRANSFORMED RAT EMBRYONIC PREADIPOSE CELL LINES"
- FEBS LETTERS, vol. 215, no. 2, pages 345-349, XP002013116 TOBE ET AL: "DIFFERENTIAL EFFECTS OF DNA TUMOR VIRUS NUCLEAR ONCOGENE PRODUCTS ON ADIPOCYTE DIFFERENTIATION"
- PROC.NATL.ACAD.SCI.USA, vol. 90, pages 9611-9615, XP002013117 NINOMIYA-TSUJI ET AL: "TUMOR NECROSIS FACTOR-INDUCED C-MYC EXPRESSION IN THE ABSENCE OF MITOGENESIS IS ASSOCIATED WITH INHIBITION OF ADIPOCYTE DIFFERENTIATION"
- J.BIOL.CHEM., vol. 265, no. 27, 25 Septembre 1990, pages 16343-16349, XP002013118 FÈVE ET AL: "DIFFERENTIAL REGULATION OF BETA1- AND BETA2-ADRENERGIC RECEPTOR PROTEIN AND MRNA LEVELS BY GLUCOCORTICOIDS DURING 3T3-F442A ADIPOSE DIFFERENTIATION"
- J.BIOL.CHEM., vol. 266, no. 30, 25 Octobre 1991, pages 20329-20336, XP002013119 FÈVE ET AL: "ATYPICAL BETA-ADRENERGIC RECEPTOR IN 3T3-F442A ADIPOCYTES.PHARMACOLOGICAL AND MOLECULAR RELATIONSHIP WITH THE HUMAN BETA3-ADRENERGIC RECEPTOR"
- J.BIOL.CHEM., vol. 269, no. 9, 4 Mars 1994, pages 6664-6670, XP002013120 KRIEF ET AL: "TRANSCRIPTIONAL MODULATION BY N-BUTYRIC ACID OF BETA1-,BETA2-, AND BETA3-ADRENERGIC RECEPTOR BALANCE IN 3T3-F442A ADIPOCYTES"
- DATABASE MEDLINE FILE SERVER STN KARLSRUHE ABR¹G¹ 94130843, KOZAK ET AL: "NOREPINEPHRINE-DEPENDENT SELECTION OF BROWN ADIPOCYTE CELL LINES" XP002013121

### LISTE DE SEQUENCES

(1) INFORMATIONS GENERALES:
   (i) DEPOSANT:
      (A) NOM: CENTRE NATIONAL DE LA RECHERCHE SCIENTIFIQUE - CNRS
      (B) RUE: 3 rue Michel Ange
      (C) VILLE: PARIS
      (E) PAYS: FRANCE
      (F) CODE POSTAL: 75794 CEDEX 16

      (A) NOM: STROSBERG Arthur Donny
      (B) RUE: 66 rue de Javel
      (C) VILLE: PARIS
      (E) PAYS: FRANCE
      (F) CODE POSTAL: 75015

      (A) NOM: ZILBERFARB Vladimir
      (B) RUE: 14 rue Le Perdriel
      (C) VILLE: VILLEBON SUR YVETTE
      (E) PAYS: FRANCE
      (F) CODE POSTAL: 91140
   (ii) TITRE DE L' INVENTION: LIGNEES IMMORTALISEES DE CELLULES ISSUES DU TISSU ADIPEUX HUMAIN, LEUR PROCEDE DE PREPARATION ET LEURS APPLICATIONS.
   (iii) NOMBRE DE SEQUENCES: 22
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
   (vi) DONNEES DE LA DEMANDE ANTERIEURE:
      (A) NUMERO DE LA DEMANDE: FR 9504922
      (B) DATE DE DEPOT: 25-APR-1995
(2) INFORMATIONS POUR LA SEQ ID NO: 1:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 1:
   TCGTGTGCAC CGTGTGGGCC 20
(2) INFORMATIONS POUR LA SEQ ID NO: 2:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 2:
   AGGAAACGGC GCTCGCAGCT GTCG 24
(2) INFORMATIONS POUR LA SEQ ID NO: 3 :
   (i) CARACTERISTIQUES DE LA SEQUENCE:
      (A) LONGUEUR: 23 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 3:
   GCCTGCTGAC CAAGAATAAG GCC 23
(2) INFORMATIONS POUR LA SEQ ID NO: 4:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 4:
   CCCATCCTGC TCCACCT 17
(2) INFORMATIONS POUR LA SEQ ID NO: 5:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 5:
   ATGGCTCCGT GGCCTCAC 18
(2) INFORMATIONS POUR LA SEQ ID NO: 6:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 6:
   CCCAACGGCC AGTGGCCAGT CAGCG 25
(2) INFORMATIONS POUR LA SEQ ID NO: 7:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 7:
   TAGGTATAAA GGTGTCCTGG 20
(2) INFORMATIONS POUR LA SEQ ID NO: 8:
   (i) CARACTERISTIQUES DE LA SEQUENCE:
      (A) LONGUEUR: 22 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 8:
   CACTTTTGTA CTGTCCTGGT GG 22
(2) INFORMATIONS POUR LA SEQ ID NO: 9:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 9:
   GAGACCTTCA ACACCCC 17
(2) INFORMATIONS POUR LA SEQ ID NO: 10:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 10:
   GTGGTGGTGA AGCTGTAG 18
(2) INFORMATIONS POUR LA SEQ ID NO: 11:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 11:
   CGAGCGAGCC AGGTGAAGCC 20
(2) INFORMATIONS POUR LA SEQ ID NO: 12:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 12:
   GCCAGCGGAA ACCTCACACG 20
(2) INFORMATIONS POUR LA SEQ ID NO: 13 :
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 13:
   TGCTGGCTGT GGGAGGA 17
(2) INFORMATIONS POUR LA SEQ ID NO: 14:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 14:
   GAGGATGCCG ACGACGAT 18
(2) INFORMATIONS POUR LA SEQ ID NO: 15:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 15:
   TCCTGCTGCC CTTCTGTC 18
(2) INFORMATIONS POUR LA SEQ ID NO: 16:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 16:
   GGCCTACCCC TGCTGTCT 18
(2) INFORMATIONS POUR LA SEQ ID NO: 17:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 17:
   CGGCTGGGGC ATAGTCA 17
(2) INFORMATIONS POUR LA SEQ ID NO: 18:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 18:
   GCACGCCCCC GCACACC 17
(2) INFORMATIONS POUR LA SEQ ID NO: 19:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 19:
   GGGGCTGAGT TTGAGCG 17
(2) INFORMATIONS POUR LA SEQ ID NO: 20:
   (i) CARACTERISTIQUES DE LA SEQUENCE:
      (A) LONGUEUR: 19 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 20:
   GCTCCTCACT GTCCTGTCC 19
(2) INFORMATIONS POUR LA SEQ ID NO: 21:
   (i) CARACTERISTIQUES DE LA SEQUENCE:
      (A) LONGUEUR: 19 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 21:
   CCTGCTCGTG CTGACTCTG 19
(2) INFORMATIONS POUR LA SEQ ID NO: 22:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "AMORCE"
   (xi) DESCRIPTION DE LA SEQUENCE: SEQ ID NO: 22:

## Description

La présente invention est relative à des lignées immortalisées de cellules issues du tissu adipeux humain, à leur procédé de préparation et à leurs applications en tant que modèle d'étude de la physiopathologie du métabolisme et notamment de l'obésité et du diabète, comme outils pour le développement de médicaments pour le traitement des états pathologiques liés aux troubles du métabolisme tels que l'obésité et le diabète et comme médicaments.

L'obésité et le diabète constituent des problèmes de santé majeurs dans le monde occidental et des facteurs de risque importants de mortalité précoce. Les facteurs considérés comme responsables du développement de l'obésité et du diabète ne sont pas encore tous bien connus. Toutefois, il est maintenant bien admis que les défauts de thermogenèse (par exemple des défauts de dissipation d'énergie sous forme de chaleur, avec comme conséquence une accumulation de lipides) interviennent dans les mécanismes de l'obésité ; il est également bien admis que le tissu adipeux et notamment le tissu adipeux brun est l'effecteur principal de la thermogenèse facultative chimique.

Le tissu adipeux (brun et blanc) est essentiellement constitué d'adipocytes (3/4 du nombre total de cellules du tissu adipeux). Les autres cellules présentes dans le tissu adipeux appartiennent notamment aux cellules précurseurs, également dénommées cellules interstitielles (cellules sans lipides) et comprennent en particulier les adipoblastes (cellules mésenchymateuses sans lipides) et les préadipocytes (cellules estérase-positives sans lipides).

Le tissu adipeux blanc (ou WAT pour *white adipose tissue*) est considéré comme un organe distinct du tissu adipeux brun (ou BAT pour *brown adipose tissue*) ; ces deux tissus adipeux se distinguent, notamment en ce que leurs cellules précurseurs sont différentes ; la présence de protéines mitochondriales découplantes (ou UCP pour *uncoupling protein*) est spécifique au BAT (tissu adipeux brun). Pour ce qui concerne plus particulièrement le tissu adipeux brun, la chronologie de la différenciation cellulaire apparaît être la suivante : cellules interstitielles, adipocytes bruns peu différenciés, adipocytes bruns matures. Les adipocytes bruns sont caractérisés par la présence de gouttelettes de triglycérides et de nombreuses mitochondries ainsi que par la présence d'une protéine particulière, la protéine découplante, précitée.

La stimulation de la prolifération et de la différenciation du tissu adipeux brun dépend notamment des récepteurs β-adrénergiques. Le récepteur β3-adrénergique humain a été caractérisé dans les dépôts adipeux blancs de l'adulte et dans le tissu adipeux brun des nouveaux-nés et des malades atteints de phéochromocytome.

Le BAT est un tissu spécialisé dans la dissipation d'énergie, sous forme de chaleur. On le trouve chez la plupart des mammifères nouveau-nés.

Il est le site majeur du stockage et de la mobilisation de l'énergie ; son rôle en tant qu'organe endocrine, autocrine et intracrine a également été établi.

Aussi bien des lignées cellulaires réalisées chez la souris [lignée murine 3T3, lignée F44-2A (Green et al., Cell, 1975, 5, 19-27 et Cell, 1976, 7, 105-113), que des cellules vasculaires stromales du tissu adipeux ont également fait l'objet d'études, dans la mesure où les préadipocytes sont présents dans le compartiment vasculaire-stromal (G. Ailhaud et al., Int. J. Obesity, 1992, **16**, (suppl. 2), S17-S21).

Une lignée de préadipocytes a également été réalisée à partir d'embryons de rat (Shigeru Yasumoto, Mol. Cell. Biol., 1984, 4(4),712-721), sur laquelle le rôle des hormones dans le processus de différenciation cellulaire a été étudié.

Toutefois, ces différentes lignées ou cellules ne sont pas adaptées à l'étude de la physiopathologie du métabolisme humain. En conséquence, il existe un besoin pour le contrôle des maladies du métabolisme et notamment de l'obésité et du diabète chez l'homme, tant du point de vue pharmacologique (modèle d'étude pharmacologique des agents lipolytiques) que du point de vue thérapeutique.

Or, jusqu'à présent, il a été impossible d'obtenir des lignées de préadipocytes humains, aptes à être maintenus en culture suffisamment longtemps, pour pouvoir d'une part, effectivement servir de modèle à l'étude d'agents lipolytiques et d'autre part être également aptes à être utilisés en tant qu'agent thérapeutique (en thérapie génique, par exemple). En effet, les expériences réalisées sur des adipocytes humains en culture primaire sont difficilement reproductibles et difficilement réalisables de manière systématique.

La Demanderesse s'est, en conséquence, donné pour but de pourvoir à des lignées de cellules, issues du tissu adipeux brun ou blanc, utiles à la fois en tant que modèle d'étude des agents lipolytiques et comme agent thérapeutique (surexpression de la protéine découplante, par exemple) et qui ne présentent pas les inconvénients des adipocytes en culture primaire.

La présente invention a pour objet des lignées cellulaires, issues du tissu adipeux, caractérisées :
- en ce qu'elles sont constituées par des préadipocytes contenant un fragment d'acide nucléique comprenant au moins un fragment immortalisant d'un oncogène viral et au moins un promoteur sélectionné dans le groupe qui comprend le promoteur dudit oncogène viral et un fragment des régions régulatrices du gène humain de la vimentine,
- en ce qu'elles expriment au moins l'une des protéines suivantes : récepteurs β1 et β2 adrénergiques, protéine découplante (UCP), transporteurs du glucose, Glut1 et Glut4, lipoprotéine lipase (LPL) et
- en ce qu'elles sont aptes à être converties en adipocytes matures, qui produisent des lipides (graisse), expriment en outre les récepteurs α2_{A} et β3 adrénergiques ainsi que le produit d'expression du gène Ob et présentent un rapport Glut4/Glut1 inversé, par rapport aux dits préadipocytes immortalisés.

Les marqueurs spécifiques des adipocytes matures (tissu adipeux brun et tissu adipeux blanc) sont notamment : Glut4, adipsine, LPL, enzyme de la lipolyse (hormone sensitive lipase : HSL), récepteurs β1-, β2- et β3-adrénergiques et produit de l'expression du gène Ob.

Selon un mode de réalisation avantageux, lesdites lignées sont issues du tissu adipeux brun.

Conformément à ce mode de réalisation avantageux desdites lignées, le fragment immortalisant est constitué par un fragment de l'oncogène T SV40, codant pour l'antigène T du virus SV40.

Également conformément à ce mode de réalisation desdites lignées, le promoteur est sélectionné parmi le promoteur de l'oncogène T SV40 et le promoteur de la vimentine et plus particulièrement le fragment d'acide nucléique du promoteur de la vimentine compris entre les bases -878 et +93 de la séquence régulatrice de la vimentine.

Selon une disposition avantageuse de ce mode de réalisation, le fragment d'acide nucléique du promoteur de la vimentine comprend les bases -830 à -539 et/ou le fragment -540 à -140, situés en amont du site CAP.

De telles lignées, dénommées PAZ 6, ont été déposées auprès de la Collection Nationale de Culture de Microorganismes (CNCM) tenue par l'INSTITUT PASTEUR, en date du 7 février 1995 sous le n° I-1531.

De telles lignées de préadipocytes humains immortalisés présentent au moins les propriétés suivantes :
- elles expriment au moins les protéines suivantes : récepteurs β1 et β2 adrénergiques, protéine découplante, Glut1 et Glut4, lipoprotéine lipase ;
- elles sont aptes à être converties en adipocytes matures :
   - qui produisent de la graisse multilobaire,
   - qui contiennent de nombreuses mitochondries,
   - qui expriment les récepteurs α2_{A} et β3 adrénergiques ainsi que le produit d'expression du gène Ob, et
   - qui présentent un rapport Glut4/Glutl inversé, par rapport auxdits préadipocytes immortalisés.

Un fragment d'acide nucléique hybride (oncogène T de SV 40 associé au promoteur de la vimentine) a été décrit (Demande de Brevet européen 90402009.6). L'introduction de ce fragment linéaire recombinant dans le noyau de cellules en cultures primaires telles que cellules musculaires, cellules épithéliales et cellules endothéliales est suivi d'une intégration et de l'expression de l'antigène grand T, induisant une prolifération cellulaire, conduisant à des lignées cellulaires permanentes.

Toutefois, les travaux de l'Art antérieur ne suggèrent pas la possibilité que des cellules de préadipocytes, issues du tissu adipeux (brun ou blanc) humain puissent effectivement être immortalisées, en gardant toutes leurs caractéristiques (marqueurs et possibilité de différenciation). En effet, aucune lignée humaine de ce type n'a jamais pu être obtenue, qu'elle soit d'origine pathologique (par exemple cancéreuse) ou expérimentale. Bien au contraire, de nombreuses tentatives (H. HAUNER et al., J. Clin. Invest., 1989, **84**, 1663-1670) d'immortalisation ont toutes échouées, ce qui distinguait, jusqu'à présent, les adipocytes humains de nombreux autres types cellulaires.

Or, de manière inattendue, les lignées de préadipocytes immortalisés, conformes à l'invention, présentent effectivement les fonctionnalités des préadipocytes humains : expression des marqueurs des préadipocytes et maturation en adipocytes dans des conditions appropriées, notamment en présence d'insuline, de glucocorticoïdes et éventuellement d'IGF-I.

En particulier, les préadipocytes immortalisés conformes à l'invention, permettent :
- l'étude des différentes étapes de maturation de la différenciation en adipocytes : apparition des marqueurs spécifiques des adipocytes tels que récepteurs α2_{A} et β3 adrénergiques et présence de gouttelettes lipidiques,
- l'analyse de l'influence de différentes conditions de culture sur la différenciation des adipocytes : insuline, glucocorticoïdes, triiodothyronine, éventuellement associée à la pioglitazone et ses dérivés, agonistes β-adrénergiques, NPY, mélatonine...,
- l'analyse du rôle du produit d'expression du gène Ob dans les adipocytes différenciés et matures selon l'invention dans la thermogénèse,
- une étude plus proche de la réalité que ne sont les cellules CHO-RAβ3, pour l'étude des récepteurs intervenant dans les processus de lipolyse, tels que les récepteurs β2 et β3 adrénergiques.

La présente invention a également pour objet un procédé d'obtention de préadipocytes immortalisés, issus de tissus adipeux (bruns ou blancs) humains, caractérisé en ce qu'il comprend la transformation de préadipocytes issus du tissu adipeux (brun ou blanc) humain par une séquence d'acide nucléique telle que définie ci-dessus.

La présente invention a également pour objet un procédé de conversion des préadipocytes conformes à l'invention en adipocytes matures, lequel procédé est caractérisé en ce qu'il comprend la mise en culture des préadipocytes immortalisés conformes à l'invention, dans un milieu contenant au moins de l'insuline.

Selon un mode de mise en oeuvre avantageux dudit procédé, ledit milieu comprend en outre au moins l'un des composés suivants : glucocorticoïde, triiodothyronine (T3), éventuellement associée à de la pioglitazone ou à ses dérivés, agonistes des récepteurs β-adrénergiques, NPY, mélatonine.

De manière avantageuse, les préadipocytes immortalisés conformes à l'invention constituent également un outil pour la sélection de substances intervenant dans l'activation de la lipolyse et/ou de la thermogenèse ; conformément à l'invention, le procédé de sélection et d'identification de substances capables de réguler la lipolyse et/ou la thermogenèse comprend :
- la mise en contact des préadipocytes immortalisés conformes à l'invention avec au moins une substance apte à les transformer en adipocytes,
- la mise en contact des adipocytes matures obtenus avec une substance agissant sur au moins un récepteur tel que les récepteurs α2_{A} ou β3 adrénergiques, les récepteurs de l'insuline et de l'IGF, les récepteurs de l'ACTH et les récepteurs du métabolisme du glucose (Glut1, Glut4) desdits adipocytes matures, en vue de la modulation de la lipolyse et/ou de la thermogenèse et
- la détection de la formation d'un complexe du type ligand-récepteur.

Un tel procédé permet donc la sélection de ligands spécifiques des différents récepteurs cités : α2_{A} ou β3 adrénergiques, récepteurs de l'insuline et de l'IGF, récepteurs de l'ACTH et récepteurs du métabolisme du glucose (Glut1, Glut4) des adipocytes matures, lesquels ligands agissent en outre sur la modulation de la lipolyse et/ou de la thermogenèse.

La présente invention a également pour objet un modèle d'étude des protéines exprimées par les adipocytes, notamment les récepteurs α2_{A} ou β3 adrénergiques, les récepteurs de l'insuline, de l'IGF, de l'ACTH et les récepteurs du métabolisme du glucose (Glut1, Glut4) humains, caractérisé en ce qu'il est constitué par des adipocytes matures obtenus à partir des préadipocytes immortalisés conformes à l'invention.

Un tel modèle permet l'étude, d'un point de vue pharmacologique, des récepteurs impliqués dans les processus de lipolyse et de thermogenèse, qui lorsqu'ils présentent des anomalies, induisent des états pathologiques tels que le diabète et/ou l'obésité. Un tel modèle, particulièrement proche de l'état physiologique humain permet l'identification de ligands, plus affins pour ces récepteurs et ainsi la mise au point de médicaments actifs dans les maladies précitées.

La présente invention a également pour objet un modèle d'étude de la régulation des gènes présents dans les adipocytes humains, caractérisé en ce qu'il est constitué par des préadipocytes immortalisés conformes à l'invention ou des adipocytes matures obtenus à partir desdits préadipocytes immortalisés.

La présente invention a, en outre, pour objet un procédé pour l'étude de l'affinité d'un récepteur exprimé par des adipocytes, pour un ou plusieurs ligands déterminés, lequel procédé comprend :
- la mise en contact des préadipocytes immortalisés conformes à l'invention, avec au moins une substance apte à les transformer en adipocytes,
- la mise en contact des adipocytes matures obtenus avec les ligands déterminés, et
- la détection d'une réaction affine entre lesdits adipocytes et lesdits ligands déterminés.

La présente invention a, également, pour objet des compositions lipolytiques, caractérisées en ce qu'elles comprennent des adipocytes matures obtenus à partir des préadipocytes immortalisés conformes à l'invention.

La présente invention a, en outre, pour objet des compositions pharmaceutiques destinées à combattre l'obésité, caractérisées en ce qu'elles comprennent des adipocytes bruns matures, obtenus à partir des préadipocytes immortalisés conformes à l'invention, éventuellement associées à au moins un véhicule pharmaceutiquement acceptable.

La présente invention a, en outre, pour objet un kit ou trousse de détection de l'affinité éventuelle d'un ligand pour une protéine exprimée par un adipocyte, caractérisé en ce qu'il comprend :
- une culture de préadipocytes conformes à l'invention,
- des moyens de conversion desdits préadipocytes en adipocytes matures, et
- un ou plusieurs ligands témoins.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :
- la figure 1 illustre les produits exprimés par les préadipocytes immortalisés conformes à l'invention,
- la figure 2 montre la différenciation des préadipocytes en culture,
- la figure 3 montre les produits exprimés par les adipocytes obtenus à partir des préadipocytes conformes à l'invention,
- la figure 4 illustre l'effet d'un certain nombre d'agonistes β sur la lipolyse.
   Il doit être bien entendu, toutefois, que ces exemples sont donnés uniquement à titre d'illustration de l'objet de l'invention, dont ils ne constituent en aucune manière une limitation.

### EXEMPLE 1 : Lignée de préadipocytes bruns humains conforme à l'invention.

### 1) Isolement de préadipocytes d'origine humaine :

Du tissu adipeux brun est prélevé sur un enfant âgé de 18 mois, atteint d'un néphroblastome.

Les cellules vasculaires stromales sont obtenues à partir de ce tissu par une digestion à la collagénase, conformément à la méthode décrite par HAUNER et al. (J. Clin. Invest., 1989, 84, 1663-1670).

Le traitement du tissu adipeux brun est réalisé comme suit :
2 mg/ml de collagénase A (Boehringer) dans un tampon KRBH (Krebs Ringer Bicarbonate) comprenant en outre 20 mg de sérum albumine bovine (BSA)/ml sont mis en contact avec ledit tissu ; la digestion est réalisée pendant 40 min à 37°C sous agitation (bain-marie 100 t/min).

Une première filtration sur nylon (diamètre des pores : 190 µm) est réalisée, puis les fragments restants sont à nouveau digérés pendant 30 min avec la même solution à base de collagénase et filtrés également sur une membrane de nylon dont le diamètre de pores est de 190 µm.

Les suspensions cellulaires obtenues sont centrifugées à 200 g pendant 10 min.

Le culot cellulaire est lavé avec un tampon Krebs Ringer contenant de la sérumalbumine bovine (BSA), puis avec le milieu de culture ; le culot contient les préadipocytes.

Les cellules sont traitées avec une solution de Gey's, afin d'éliminer les hématies.

Les cellules obtenues (préadipocytes) sont ensemencées dans des boîtes de Pétri de 35 mm de diamètre contenant 2 ml d'un milieu de culture comprenant : DMEM/HAM F12 (1:1 vol/vol) contenant : pénicilline 100 U/ml, streptomycine 0,1 mg/ml, sérum de veau à 7,5 %, sérum de veau de nouveau-né à 2,5 % (Boehringer).

La densité d'ensemencement est de 20 000 cellules/cm².

Les cultures sont maintenues à 37°C pendant 16 heures, dans une atmosphère contenant 10 % de CO₂ et 90 % d'air.

Après adhésion, les cellules sont lavées dans un tampon PBS et le milieu de culture est renouvelé et est constitué de 2 ml d'un milieu ITT (DMEM/Ham's F12 (1:1, v/v) et glutamax I, contenant 33 µM de biotine, 17 µM de pantothénate, 15 mM d'hépès, 0,2 nM de triiodothyronine (T3), de la pénicilline à 100 U/ml, de la streptomycine à 0,1 mg/ml), du sérum de veau à 7,5 % et du sérum de veau nouveau né à 2,5 %.

Le milieu est changé tous les 2 jours.

### 2) Immortalisation :

Les cellules ainsi obtenues sont transfectées par microinjection avec une construction comprenant la séquence codant pour l'antigène grand T de SV40 sous le contrôle du promoteur de la vimentine (Hu Vim 830-T/t).

Le plasmide contenant cette séquence est décrit dans la Demande de Brevet européen n° 90402009.6 ; il exprime l'antigène T et l'antigène t du virus SV40 et est obtenu comme suit :

Le fragment -830-+93 du promoteur de la vimentine est cloné dans un plasmide pUC18 ; les clones sont obtenus par digestion de la séquence 5' du gène de la vimentine au niveau du site de l'enzyme de restriction PvuII.

Le plasmide pUC18 contenant le fragment d'ADN du promoteur de la vimentine est linéarisé par l'enzyme de restriction XbaI ; on obtient une extrémité 3' cohésive, qui est ajustée de manière à obtenir une extrémité franche, puis on lie le fragment d'ADN du promoteur de la vimentine ainsi obtenu avec le fragment d'ADN de l'antigène T, par ligation de l'extrémité 3' XbaI du promoteur avec l'extrémité SfiI du fragment SV40.

Les extrémités BamHI 5' et 3' sont liées en présence de T4 ligase.

Les séquences codant pour l'antigène T sont ainsi sous le contrôle de la région régulatrice de la vimentine de 830 paires de bases.

Les cellules ainsi transfectées sont cultivées sur le même milieu, sans T3 ; lorsqu'elles atteignent la confluence, elles sont trypsinisées une fois par semaine, de manière à permettre un passage hebdomadaire.

On obtient ainsi la lignée cellulaire de préadipocytes immortalisés, dénommée PAZ-6.

### 3) Caractéristiques de la lignée cellulaire de préadipocytes immortalisés PAZ-6 :

Les préadipocytes bruns immortalisés, dénommés PAZ-6, peuvent être mis en culture (passage hebdomadaire) pendant plusieurs mois sans perdre leurs caractéristiques morphologiques ou leurs marqueurs moléculaires qui peuvent être identifiés par PCR.
- différenciation en adipocytes matures :

A n'importe quel moment, les préadipocytes bruns immortalisés obtenus peuvent être convertis en adipocytes par traitement avec de l'insuline et de la dexaméthasone ; cette conversion est caractérisée par l'accumulation de graisses multilobaires et par l'expression des marqueurs spécifiques des adipocytes que sont les récepteurs α2_{A} et β3 adrénergiques, ainsi que l'expression de la lipoprotéine lipase.

Les conditions de la conversion sont les suivantes :
les cellules sont ensemencées à une densité de 10 000 cellules/cm² et cultivées pendant 3 jours dans un milieu contenant du 6 % de sérum de veau foetal, de manière à obtenir la confluence ; on additionne ensuite aux dites cellules un milieu ITT (milieu DMEM/HAM F12 supplémenté avec de la biotine, du pantothénate, de l'Hépès, de la T3, de la pénicilline et de la streptomycine, tel que défini ci-dessus), lequel milieu contient, en outre, 0,1 µM de dexaméthasone, 850 nM d'insuline, 1 µM de pioglitazone, 0,25 mM d'IBMX (3-isobutyl-1-méthyl-xanthine) pendant 4 jours. Le milieu de culture est changé tous les 2 jours ; la différenciation est obtenue en environ deux semaines comme suit : après 15 à 21 jours dans ces conditions de culture, les cellules sont lavées avec un tampon PBS et remises en culture dans un milieu ne contenant ni insuline, ni dexaméthasone, ni sérum, mais supplémenté avec de la transferrine à 10 µg/ml et de la T3 à 1 nM pendant 24 heures avant d'utiliser ces cellules différenciées.

La vitesse de conversion est accélérée en présence de pioglitazone : en effet, les préadipocytes PAZ-6, en présence d'insuline et de dexaméthasone sont différenciées en adipocytes matures capables d'accumuler des globules de graisse et d'effectuer une lipolyse ; toutefois, cette différenciation est significativement augmentée en présence de pioglitazone.

### a. Marqueurs des préadipocytes bruns immortalisés et des adipocytes matures obtenus à partir de ces préadipocytes :

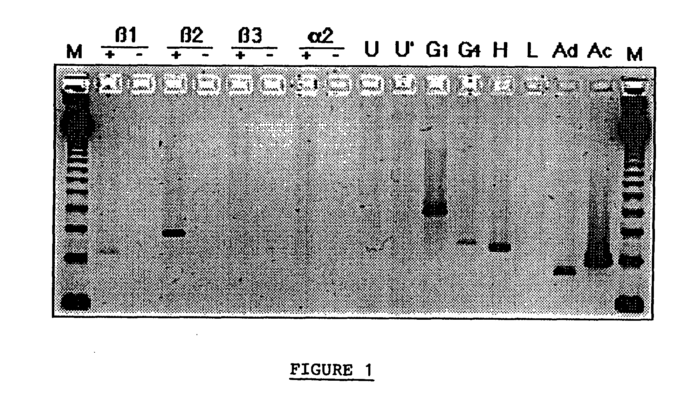

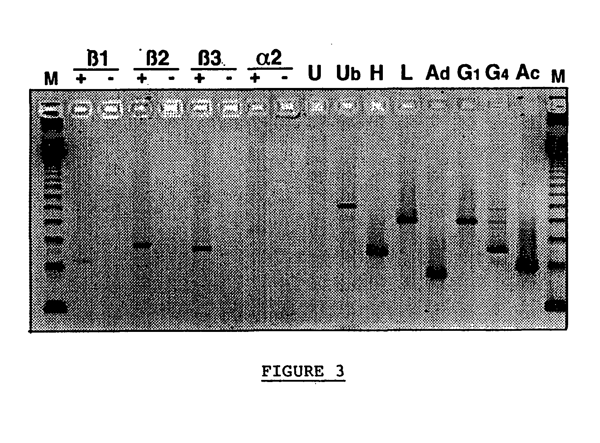

récepteurs β1 et β2 adrénergiques, protéine découplante (UCP), GLUT1 et GLUT4 (voir figure 1) ; marqueurs spécifiques des adipocytes matures obtenus : récepteurs α2_{A} et β3 adrénergiques et lipoprotéine lipase (figure 3) ; en outre, la présence de nombreuses mitochondries confirme que ces cellules sont des adipocytes bruns matures.

### - Matériel et méthode mis en oeuvre :

Après différenciation, les adipocytes matures sont cultivés 24 à 48 heures dans le milieu ITT supplémenté avec 10 µg/ml de transferrine.

Les conditions opératoires sont les suivantes :

L'ARN total des cellules PAZ-6 à analyser (préadipocytes immortalisés ou adipocytes matures) est extrait (CATHALA, 1983, DNA, 2, 329-335) et traité pendant **20** min à 37°C avec 0,3 U de DNase I sans RNase (RQ1 DNase, Promega) par µg d'acide nucléique, dans un milieu comprenant 40 mM de tampon Tris-HCl à pH 7,9, 10 mM de NaCl et 6 mM de MgCl₂ en présence de 2 U/µl d'inhibiteur de RNase placentaire (RNasin, Promega). L'ARN est ensuite extrait par un mélange phénol/chloroforme et précipité.

La synthèse de l'ADNc est effectuée avec 100 U de transcriptase inverse de MLV (Gibco BRL) avec 200 ng d'ARN DNasé dans 10 µl de tampon de transcriptase inverse (50 mM Tris-HCl pH 8,3, 75 mM KCl, 3 mM MgCl₂ et 10 mM DTT) contenant 0,4 mM de chaque dNTP, 10 µm d'hexanucléotides aléatoires (Pharmacia, France) et 2 U/µl d'inhibiteur de RNase placentaire. Pour les gènes sans introns, un contrôle sans transcriptase inverse a été effectué pour vérifier que l'amplification ne résulte pas d'ADN génomique résiduel.

Les échantillons sont ensuite supplémentés avec 4 µl de tampon 10xPCR (tampon 1xPCR : 50 mM KCl, 10 mM Tris-HCl pH 9,0 à 25°C, 0,1 % Triton X-100 et 1,5 mM MgCl₂), 0,5 µl de chaque dNTP 25 mM, 10 µl de DMSO à 50 % (pour les récepteurs β1, β2, β3, α2 et l'adipsine), 0,5 µl d'oligonucléotides sens et antisens à 25 mM chacun, 1 U de Taq-ADN polymérase (Promega) et de l'eau jusqu'à 40 µl.

Les ADNc sont dénaturés pendant 2 minutes à 94°C et amplifiés avec 29 cycles de températures (94°C, 15 s ; 60°C, 30 s ; 72°C, 30 s) suivis par 3 minutes d'extension finale à 72°C dans un thermocycleur (GeneAmpPCR System 9600, Perkin-Elmer Cetus).

Les séquences des oligonucléotides sens et antisens et la taille des produits d'amplification sont les suivantes :
- RA-β1 : taille du produit amplifié : 265 pb ;
- RA-β2 : taille du produit amplifié : 329 pb ;
- RA-β3 : taille du produit amplifié : 316 pb (KRIEF, J. Clin. Invest., 1993, 91, 344-349).
- UCP : taille du produit amplifié : 590 pb (CASSARD et al., J. Cell Biochem., 1990, 43, 255-264).
   Selon le type d'amplification on obtient deux produits :
   U = 29 cycles PCR
   U' = 39 cycles PCR.
- β-actine : taille du produit amplifié : 236 pb (FEVE et al., J. Biol. Chem., 1992, 267, 15909-15915) ;
- α2-A : taille du produit amplifié : 403 pb (KOBILKA et al., Science, 1987, 238, 650-656) ;
- GLUT1 : taille du produit amplifié : 470 pb (MUECKLER et al., Science, 1985, 229, 941-945) ;
- GLUT4 : taille du produit amplifié : 309 pb (FUKUMOTO & al., J. Biol. Chem., 1989, 264, 7776-7779) ;
- adipsine : taille du produit amplifié : 200 pb (WHITE et al., J. Biol. Chem., 1992, 267, 9210-921) ;
- lipase (hormono-sensible) (HSL) : taille du produit amplifié : 286 pb (LANGIN et al., Proc. Natl. Acad. Sci. USA, 1993, 90, 4897-4901) ;
- lipase (lipoprotéine) : taille du produit amplifié : 473 pb (WION KL. et al., Science, 1987, 235, 1638-1641).

Les séquences nucléotidiques subséquentes ont toutes été déterminées en employant le programme d'analyse élémentaire Oligo 4.0 (National Biosciences, Plymouth, MN 5547, USA).

En RT-PCR, les mêmes ARN messagers sont identifiés dans le clone et dans la lignée.

### - Résultats :

La figure 1 illustre les produits exprimés par les préadipocytes immortalisés conformes à l'invention (lignée PAZ-6) : ces préadipocytes expriment les récepteurs adrénergiques β1 (265 pb), β2 (329 pb) et α2_{A} (403 pb), UCP (protéine découplante) (U et U' : 590 pb), HSL (Hormone Sensitive Lipase) (H) (286 pb), adipsine (Ad) (200 pb), GLUT1 (G1) (470 pb), GLUT4 (G4) (309 pb) et β-actine (Ac) (336 pb). Chaque trace correspond à 200 ng d'ARN total initial, à partir duquel l'ADNc a été amplifié comme suit : RT-PCR de 29 cycles (sauf U', PCR de 39 cycles) effectuée avec (+) ou sans (-) reverse transcriptase.

M correspond à l'échelle de marqueur 123 pb.

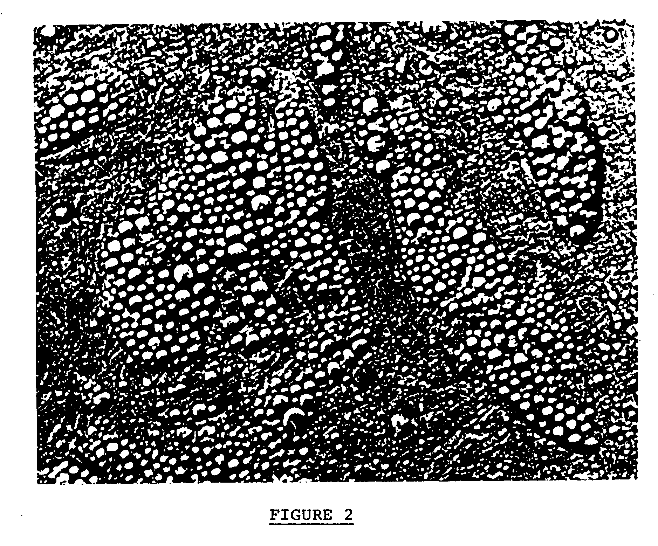

La figure 2 montre la différenciation des pré-adipocytes en culture après confluence dans un milieu ITT supplémenté en T3, insuline et dexaméthasone, pendant 3 semaines.

La figure 3 montre les marqueurs exprimés par les adipocytes et notamment les récepteurs β3 (β3, 316 pb) et α2_{A} adrénergiques et la LPL ; en outre les niveaux d'expression d'adipsine, de HSL, LPL et GLUT4 sont plus importants que ceux des préadipocytes.

En outre, ces cellules différenciées expriment encore l'UCP (figure 3). L'ARNm de l'UCP est également présent par hybridation *Northern.*

2 semaines après la conversion en adipocytes, les cellules sont mises en culture dans un milieu dépourvu d'insuline et de dexaméthasone, ce qui dans les autres types cellulaires précédemment utilisés entraînait une diminution de l'expression du récepteur adrénergique β3, alors que ce n'est pas le cas pour ce qui concerne les cellules PAZ-6 selon l'invention.

La RT-PCR est réalisée dans les mêmes conditions que celles de la figure 1 (Ub=U'). 5 µl sur 50 µl total ont été déposé sur gel. L'image du gel est en négatif. Les mêmes fragments sont amplifiés.

Comme illustré ci-dessus, le RA-β3 est exprimé lorsque les préadipocytes bruns humains sont convertis en adipocytes après traitement avec l'insuline, la dexaméthasone et la pioglitazone.

Cette expression est également observée au niveau ARNm à l'aide d'une amplification PCR modérée (29 cycles) et au niveau du récepteur à l'aide des essais de liaison en présence d'antagonistes β1 et β2 (voir b.).

### b. mise en évidence des récepteurs adrénergiques β (RA-β).

### * détermination des sites de liaison au ligand antagoniste β, l'iodocyanopindolol (I-CYP), dans la lignée cellulaire PAZ-6.

### - Méthode :

Les cellules sont lavées avec un tampon PBS, incubées pendant 5 minutes à 37°C avec une solution de trypsine/EDTA à 2 % et remises en suspension dans un milieu DMEM supplémenté avec du sérum de cheval à 10 % (v/v).

Après centrifugation à 450 x g pendant 5 minutes à 4°C, le culot cellulaire est lavé 2 fois avec un tampon PBS, puis les cellules sont remises en suspension dans du tampon PBS.

Pour les essais de liaison, 100 µl de suspension cellulaire sont incubés (1) en présence de 500 pM d'I-CYP, et en présence ou en l'absence de bupranolol 50 µM (pour définir les liaisons non-spécifiques) ou (2) en présence de ICI 118551 et/ou de CGP 20712A (chacun à 0,1 mM).

L'essai de liaison proprement dit est effectué pendant 90 min à 25°C, dans un volume final de 250 ml de PBS supplémenté avec 50 mg/ml de sérum albumine bovine et de désipramine 1 mM.

La réaction est stoppée à l'aide d'une filtration rapide à travers un filtre en fibre de verre Whatman GF/C, préalablement immergé dans une solution de PBS contenant de la polyéthylèneimine à 0,3 %, pendant 30 min (pour réduire les liaisons non-spécifiques).

Les concentrations en protéine sont déterminées selon la méthode de M. Bradford (Anal. Biochem., 1976, 72, 248-256).

La sérum albumine bovine est utilisée comme standard et les suspensions cellulaires sont homogénéisées avec un homogénéiseur polytron pendant 5 secondes au maximum, avant la réaction.

### - Résultats :

Dans la lignée non différenciée PAZ-6, environ 30 fmol de sites de liaison au I-CYP, dans les membranes isolées sont complètement bloqués par 50 µM de bupranolol (la quantité de sites au I-CYP est donc d'environ 70 fmol), en présence de 500 pM de I-CYP et de 0,05 mM de bupranolol (qui bloque tous les sites de liaison β-spécifiques : β1-, β2 et β3 RA), comme illustré au Tableau I ci-après.

En présence de 100 nM d'ICI 118551 (antagoniste spécifique des Raβ2), les sites de liaison au I-CYP sont diminués de 46 %.

La présence de sites RA-β1 est vérifiée en utilisant du CGP 20712A, antagoniste spécifique des RA-β1, à une concentration de 10 nM. Dans ces conditions, 58 % des sites de liaison au I-CYP sont inhibés. Les deux antagonistes, utilisés simultanément, entraînent une diminution (inhibition) du nombre de sites de liaison de 86 %.

Ceci indique que les sites de liaison au ICYP dans les cellules PAZ-6 non différenciées sont pour la plupart des sites β1- et β2-adrénergiques, en quantités équimolaires.

Après différenciation de la lignée PAZ-6, on détermine la présence d'environ 20 fmol de sites de liaison au I-CYP, dans les mêmes conditions que celles exposées ci-dessus. En présence de l'antagoniste spécifique des RA-β2, ICI 118551, le nombre de sites de liaison est réduit de 34 %. En présence de l'antagoniste spécifique des RA-β1, CGP 20712A, une diminution de 35 % est observée. Les deux antagonistes, utilisés simultanément, diminuent le nombre de sites de liaison de 64 %.

Ce résultat indique la présence de sites β1- et β2-adrénergiques en quantités équimolaires ainsi que la présence de sites β3, qui constituent au moins 36 % du nombre total de sites de liaison au I-CYP.

**TABLEAU I**

| Accumulation d'AMPc | | | | | | |
|---|---|---|---|---|---|---|
| type cellulaire | Différenciation | pmol d'AMPc/mg prot | | | facteur de multiplication/taux basal | |
| | | basal | ISO | CGP12177A | ISO | CGP12177A |
| PAZ-6 | non | 201±30 | 4344±1060 | 254±167 | 21±2 | 1,3±1 |
| PAZ-6 | oui | 130±26 | 2083±1420 | 323±129 | 15±7 | 2,4±0,5 |

| | | | | | | |
|---|---|---|---|---|---|---|
| ISO = isoprénaline. | | | | | | |

### * accumulation d'AMPc dans la lignée cellulaire PAZ-6.

Dans la mesure où il n'existe pas d'antagoniste spécifique des RA-β3, une preuve directe de la présence de RA-β3 ne peut pas être obtenue à partir de la détermination des sites de liaison au I-CYP. Toutefois, il existe un agoniste partiel, spécifique des RA-β3 (CGP12177A), qui antagonise les RA-β1 et β2 ; en conséquence, une accumulation d'AMPc, après stimulation avec le CGP12177A, donnera une preuve directe de la présence de RA-β3.

### - Méthode :

La méthode utilisée pour déterminer les taux intracellulaires d'AMPc est la suivante :
les cellules sont lavées une fois dans du PBS et incubées en l'absence ou en présence de 10 mM d'isoprénaline ou de CGP 12177A, pendant 15 min à 37°C, dans un tampon comprenant du PBS, de l'IBMX 0,5 mM et de l'acide ascorbique 0,5 mM.

Le tampon d'incubation est éliminé et les cellules sont lysées dans NaOH 1 M pendant au moins 20 min à 37°C. Le lysat est neutralisé avec de l'acide acétique 1 M et centrifugé dans une microcentrifugeuse, à vitesse maximale, pendant 5 min.

Le surnageant est utilisé pour la détermination de l'AMPc (kit AMPc-³H Amersham).

### - Résultats :

La stimulation des cellules PAZ-6 non-différenciées, par de l'isoprotérénol (10 µM), active tous les récepteurs adrénergiques β, entraînant une augmentation des taux d'AMPc d'un facteur 21. CGP 12177A (antagoniste β1 et β2 et agoniste partiel β3) n'entraîne aucune stimulation significative de l'adénylyl cyclase, ce qui confirme l'absence de récepteurs adrénergiques β3 fonctionnels dans ces cellules PAZ-6 non-différenciées.

La stimulation de la lignée cellulaire PAZ-6 différenciée avec de l'isoprotérénol (10 µM) active tous les RA-β (augmentation du taux d'AMPc/taux basal, d'un facteur 15), tandis que la stimulation avec du CGP 12177A entraîne seulement une stimulation de 20 % (augmentation du taux d'AMPc d'un facteur 2,4), suggérant la présence d'un nombre significatif de récepteurs adrénergiques β3 couplés à l'adénylyl cyclase).

Cette stimulation partielle peut s'expliquer par la présence de RA-β1 et -β2, qui ne sont pas stimulés par le CGP 12177A et par les propriétés agonistes partielles de ce composé. Ce résultat est compatible avec la conclusion ci-dessus qui précise que les sites de liaison au I-CYP, qui ne peuvent pas être bloqués par les antagonistes des RA-β1 et -β2 mais peuvent être bloqués par le bupranolol, représentent des sites de liaison aux RA-β3.

### c. Essai de lipolyse (sur la lignée PAZ-6) :

### - Méthode :

Le test de lipolyse consiste à mesurer la quantité de glycérol généré par les adipocytes matures traités par des ligands lipolytiques.

Des monocouches d'adipocytes matures (différenciés) en plaques de culture (6 puits) sont lavées .et préincubées une nuit dans du DMEM/Ham's F12 (1:1, v/v) comprenant 1 % de BSA sans acide gras. Les cellules sont ensuite incubées dans un tampon Krebs-Ringer phosphate (pH 7,4) comprenant 2 % de BSA sans acide gras, 4,5 g/l de glucose, 50 µg/ml de Na₂S₂O₅ et les ligands β-adrénergiques, dans un volume de 500 µl, pendant 2 heures à 37°C.

Le taux de glycérol est mesuré sur 100 µl de milieu d'incubation en suivant la réduction du NAD en NADH à 340 nm en présence de 10 µg/ml de glycérol déshydrogénase (issu d'*Enterobacter aerogenes*, Boehringer Mannheim) dans le tampon de réaction suivant : K₂CO₃/NaHCO₃ 125 mM pH 10, NAD 3,5 mM et (NH₄)₂SO₄ 330 mM.

### - Résultats :

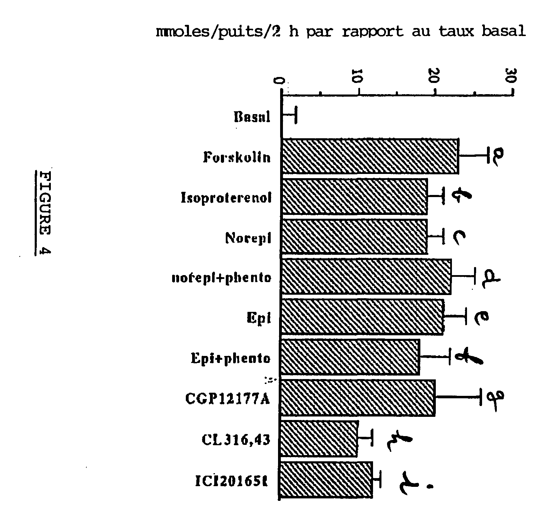

Les résultats sont reportés à la figure 4 qui illustre les résultats obtenus pour différents agonistes β: forskoline 10⁻⁴ M (colonne a), isoprotérénol 10⁻⁵ M (colonne b), norépinéphrine 10⁻⁵ M (colonne c), mélange norépinéphrine 10⁻⁵ M + phentolamine à 20 µg/ml (colonne d), épinéphrine 10⁻⁵ M (colonne e), mélange épinéphrine 10⁻⁵ M + phentolamine à 20 µg/ml (colonne f), CGP 12177A 10⁻⁵ M (colonne g), CL 316,43 10⁻⁵ M (colonne h), ICI 201651 10⁻⁵ M (colonne i), exprimés en nmol/puits/2 heures par rapport au taux basal.

Les résultats, illustrés à la figure 4, montrent une lipolyse induite par un agent stimulant directement l'adénylyl cyclase (forskoline), par des agonistes des RA-β1 et RA-β2 (isoprotérénol, norépinéphrine et épinéphrine), en présence ou non d'un antagoniste α2 adrénergique (phentolamine). On observe également une lipolyse induite par les agonistes spécifiques du RA-β3 (antagoniste RA-β1 et RA-β2, comme le CGP 12777A, le CL 316,43 et l'ICI 20651). Ces observations suggèrent que la plupart de la lipolyse induite par l'isoprotérénol dans les adipocytes humains différenciés PAZ-6, est contrôlée par les récepteurs adrénergiques β3.

### d. Expression du gène Ob dans les cellules PAZ-6 différenciées.

Les cellules PAZ-6 différenciées expriment l'équivalent humain du produit du gène Ob murin.

Lorsqu'elle est injectée, la leptine (protéine Ob) diminue apparemment l'appétit et par voie de conséquence l'obésité chez les souris Ob, dans lesquelles le gène correspondant est muté. Chez l'Homme, aucune mutation du gène Ob n'a été observée et la concentration de la protéine Ob est significativement augmentée chez les sujets obèses.

L'apparition simultanée du RA-β3 et du produit du gène Ob dans les adipocytes matures et différenciés PAZ-6, suggère l'existence d'un lien entre ces deux protéines.

En particulier, le traitement d'adipocytes de rat avec l'agoniste β3 CL 316,43, semble réduire de manière importante les taux d'ARNm codant pour la protéine Ob.

Le fait que le gène Ob soit exprimé dans les adipocytes PAZ-6 est tout à fait surprenant. En effet, jusqu'à présent la protéine Ob a été essentiellement considérée comme jouant un rôle dans la régulation de la satiété. Sa mise en évidence dans les adipocytes bruns suggère qu'elle joue un rôle dans la thermogénèse.

## Revendications

1. Lignées cellulaires, issues du tissu adipeux humain, **caractérisées :**
- **en ce qu'**elles sont constituées par des préadipocytes humains contenant un fragment d'acide nucléique comprenant au moins un fragment immortalisant d'un oncogène viral et au moins un promoteur sélectionné dans le groupe qui comprend le promoteur dudit oncogène viral et un fragment des régions régulatrices du gène humain de la vimentine,
- **en ce qu'**elles expriment au moins l'une des protéines suivantes : récepteurs β1 et β2 adrénergiques, protéine découplante (UCP), transporteurs de glucose, Glutl et Glut4, lipoprotéine lipase (LPL) et
- **en ce qu'**elles sont aptes à être converties en adipocytes humains matures, qui produisent de la graisse, qui contiennent de nombreuses mitochondries, et expriment en outre les récepteurs α2_{A} et β3 adrénergiques ainsi que le produit d'expression du gène Ob et présentent un rapport Glut4/Glut1 inversé, par rapport auxdits préadipocytes humains immortalisés.

2. Lignées cellulaires selon la revendication 1, **caractérisées en ce qu'**elles sont issues du tissu adipeux brun.

3. Lignées cellulaires selon la revendication 1 ou la revendication 2, **caractérisées en ce que** le fragment immortalisant est constitué par un fragment de l'oncogène T SV40, codant pour l'antigène T du virus SV40.

4. Lignées cellulaires selon l'une quelconque des revendications 1 à 3, **caractérisées en ce que** le promoteur est sélectionné parmi le promoteur de l'oncogène T SV40 et le promoteur de la vimentine et plus particulièrement le fragment d'acide nucléique du promoteur de la vimentine compris entre les bases -878 et +93 de la séquence régulatrice de la vimentine.

5. Lignées cellulaires selon la revendication 4, **caractérisées en ce que** le fragment d'acide nucléique du promoteur de la vimentine comprend les bases -830 à -539 et/ou le fragment -540 à -140, situés en amont du site CAP.

6. Lignées cellulaires selon la revendication 4 ou la revendication 5, **caractérisées en ce qu'**elles ont été déposées auprès de la Collection Nationale de Culture de Microorganismes (CNCM) tenue par l'INSTITUT PASTEUR, en date du 7 février 1995 sous le n° I-1531.

7. Lignées cellulaires selon l'une quelconque des revendications 2 à 6, issues du tissu adipeux brun, **caractérisées :**
- **en ce qu'**elles expriment au moins les protéines suivantes : récepteurs β1 et β2 adrénergiques, protéine découplante, Glutl et Glut4, lipoprotéine lipase ;
- **en ce qu'**elles sont aptes à être converties en adipocytes matures :
. qui produisent de la graisse multilobaire,
. qui contiennent de nombreuses mitochondries,
. qui expriment les récepteurs α2_{A} et β3 adrénergiques ainsi que le produit d'expression du gène Ob, et
. qui présentent un rapport Glut4/Glut1 inversé, par rapport aux dits préadipocytes immortalisés.

8. Procédé d'obtention de préadipocytes immortalisés, issus de tissus adipeux (bruns ou blancs) humains, **caractérisé en ce qu'**il comprend la transformation de préadipocytes issus du tissu adipeux (brun ou blanc) humain par une séquence d'acide nucléique comprenant au moins un fragment immortalisant d'un oncogène viral et au moins un promoteur sélectionné dans le groupe qui comprend le promoteur dudit oncogène viral et un fragment des régions régulatrices du gène humain de la vimentine telle que définie à l'une quelconque des revendications 1 à 7.

9. Procédé de conversion des préadipocytes selon l'une quelconque des revendications 1 à 7 en adipocytes matures, lequel procédé est **caractérisé en ce qu'**il comprend la mise en culture des préadipocytes immortalisés selon l'une quelconque des revendications 1 à 7.

10. Procédé selon la revendication 9, **caractérisé en ce que** ledit milieu comprend en outre au moins l'un des composés suivants : glucocorticoïde, triiodothyronine (T3), éventuellement associée à de la pioglitazone ou à ses dérivés, agonistes des récepteurs β-adrénergiques, NPY, mélatonine.

11. Procédé de sélection et d'identification de substances capables de réguler la lipolyse et/ou la thermogenèse, **caractérisé en ce qu'**il comprend :
- la mise en contact des préadipocytes immortalisés selon l'une quelconque des revendications 1 à 7, avec au moins une substance apte à les transformer en adipocytes,
- la mise en contact des adipocytes matures obtenus avec une substance agissant sur au moins un récepteur tels que les récepteurs α2_{A} ou β3 adrénergiques, les récepteurs de l'insuline, de l'IGF, les récepteurs de l'ACTH et les récepteurs du métabolisme du glucose (Glut1, Glut4) desdits adipocytes matures, en vue de la modulation de la lipolyse et/ou de la thermogenèse et
- la détection de la formation d'un complexe du type ligand-récepteur.

12. Modèle d'étude des protéines exprimées par les adipocytes, **caractérisé en ce qu'**il est constitué par des préadipocytes immortalisés selon l'une quelconque des revendications 1 à 7 ou des adipocytes matures obtenus à partir desdits préadipocytes immortalisés.

13. Compositions lipolytiques, **caractérisées en ce qu'**elles comprennent des adipocytes matures obtenus à partir des préadipocytes immortalisés selon l'une quelconque des revendications 1 à 7.

14. Modèle d'étude de la régulation des gènes présents dans les adipocytes humains, **caractérisé en ce qu'**il est constitué par des préadipocytes immortalisés selon l'une quelconque des revendications 1 à 7 ou des adipocytes matures obtenus à partir desdits préadipocytes immortalisés .

15. Compositions pharmaceutiques destinées à combattre l'obésité, **caractérisées en ce qu'**elles comprennent des adipocytes bruns matures, obtenus à partir des préadipocytes immortalisés selon l'une quelconque des revendications 1 à 7, éventuellement associées à au moins un véhicule pharmaceutiquement acceptable.

16. Procédé pour l'étude de l'affinité d'un récepteur exprimé par des adipocytes, pour un ou plusieurs ligands déterminés, lequel procédé comprend :
- la mise en contact des préadipocytes immortalisés selon l'une quelconque des revendications 1 à 7, avec au moins une substance apte à les transformer en adipocytes,
- la mise en contact des adipocytes matures obtenus avec les ligands déterminés, et
- la détection d'une réaction affine entre lesdits adipocytes et lesdits ligands déterminés.

17. Kit ou trousse de détection de l'affinité éventuelle d'un ligand pour une protéine exprimée par un adipocyte, **caractérisé en ce qu'**il comprend :
- une culture de préadipocytes selon l'une quelconque des revendications 1 à 7,
- des moyens de conversion desdits préadipocytes en adipocytes matures, et
un ou plusieurs ligands témoins.

## Claims

1. Cell lines from human adipose tissue, **characterized in that**:
- they consist of human preadipocytes containing a nucleic acid fragment comprising at least one immortalizing fragment of a viral oncogene and at least one promoter selected from the group comprising the promoter of said viral oncogene and a fragment of the regulatory regions of the human vimentin gene,
- they express at least one of the following proteins: β1- and β2-adrenergic receptors, uncoupling protein (UCP), glucose transporters Glut1 and Glut4, and lipoprotein lipase (LPL), and
- they are capable of being converted to mature human adipocytes which produce fat, contain numerous mitochondria, additionally express the α2_{A}- and β3-adrenergic receptors as well as the expression product of the Ob gene, and have an inverted ratio Glut4/Glut1 relative to said immortalized human preadipocytes.

2. Cell lines according to claim 1, **characterized in that** they are derived from brown adipose tissue.

3. Cell lines according to claim 1 or claim 2, **characterized in that** the immortalizing fragment consists of a fragment of the SV40 T oncogene coding for the T antigen of the SV40 virus.

4. Cell lines according to any one of claims 1 to 3, **characterized in that** the promoter is selected from the SV40 T oncogene promoter and the vimentin promoter, more particularly the nucleic acid fragment of the vimentin promoter between bases -878 and +93 of the regulatory sequence of vimentin.

5. Cell lines according to claim 4, **characterized in that** the nucleic acid fragment of the vimentin promoter comprises bases -830 to -539 and/or the fragment -540 to -140, located upstream of the CAP site.

6. Cell lines according to claim 4 or claim 5, **characterized in that** they were deposited on 7^{th} February 1995 under no. I-1531 in the Collection Nationale de Culture de Microorganismes (CNCM) held by the INSTITUT PASTEUR.

7. Cell lines according to any one of claims 2 to 6, derived from brown adipose tissue, **characterized in that**:
- they express at least the following proteins: β1- and β2-adrenergic receptors, uncoupling protein, Glut1 and Glut4, lipoprotein lipase; and
- they are capable of being converted to mature adipocytes which:
. produce multilobar fat,
. contain numerous mitochondria,
. express the α2_{A}- and β3-adrenergic receptors and the expression product of the Ob gene, and
. have an inverted ratio Glut4/Glut1 relative to said immortalized preadipocytes.

8. Process for the preparation of immortalized preadipocytes from human (brown or white) adipose tissues, **characterized in that** it comprises converting preadipocytes from human (brown or white) adipose tissue by means of a nucleic acid sequence comprising at least one immortalizing fragment of a viral oncogene and at least one promoter selected from the group comprising the promoter of said viral oncogene and a fragment of the regulatory regions of the human vimentin gene, as defined in any one of claims 1 to 7.

9. Process for the conversion of the preadipocytes according to any one of claims 1 to 7 to mature adipocytes, which process is **characterized in that** it comprises the cultivation of the immortalized preadipocytes according to any one of claims 1 to 7.

10. Process according to claim 9, **characterized in that** said medium also comprises at least one of the following compounds: glucocorticoid, triiodothyronine (T3) optionally associated with pioglitazone or its derivatives, β-adrenergic receptor agonists, NPY and melatonin.

11. Process for the selection and identification of substances capable of regulating lipolysis and/or thermogenesis, **characterized in that** it comprises:
- bringing the immortalized preadipocytes according to any one of claims 1 to 7 into contact with at least one substance capable of converting them to adipocytes,
- bringing the mature adipocytes obtained into contact with a substance which acts on at least one receptor of said mature adipocytes, such as the α2_{A}- or β₃-adrenergic receptors, the insulin and IGF receptors, the ACTH receptors and the glucose metabolism receptors (Glut1, Glut4), in order to modify lipolysis and/or thermogenesis, and
- detecting the formation of a complex of the ligand-receptor type.

12. Model for studying the proteins expressed by adipocytes, **characterized in that** it consists of immortalized preadipocytes according to any one of said claims 1 to 7, or mature adipocytes obtained from said immortalized preadipocytes.

13. Lipolytic compositions, **characterized in that** they comprise mature adipocytes obtained from the immortalized preadipocytes according to any one of claims 1 to 7.

14. Model for studying the regulation of the genes present in human adipocytes, **characterized in that** it consists of immortalized preadipocytes according to any one of claims 1 to 7, or mature adipocytes obtained from said immortalized preadipocytes.

15. Pharmaceutical compositions for controlling obesity, **characterized in that** they comprise mature brown adipocytes obtained from the immortalized preadipocytes according to any one of claims 1 to 7, optionally in association with at least one pharmaceutically acceptable vehicle.

16. Process for studying the affinity of a receptor expressed by adipocytes for one or more given ligands, which process comprises:
- bringing the immortalized preadipocytes according to any one of claims 1 to 7 into contact with at least one substance capable of converting them to adipocytes,
- bringing the mature adipocytes obtained into contact with the given ligands, and
- detecting an affinity reaction between said adipocytes and said given ligands.

17. Kit for detecting the possible affinity of a ligand for a protein expressed by an adipocyte, **characterized in that** it comprises:
- a culture of preadipocytes according to any one of claims 1 to 7,
- means of converting said preadipocytes to mature adipocytes, and
- one or more control ligands.

## Patentansprüche

1. Zellinien, die aus humanem Fettgewebe stammen, **dadurch gekennzeichnet:**
- **dass** sie aus humanen Präadipozyten bestehen, die ein Nukleinsäurefragment enthalten, das wenigstens ein immortalisierendes Fragment eines viralen Onkogens und wenigstens einen Promotor umfasst, der ausgewählt ist aus der Gruppe, die den Promotor dieses viralen Onkogens und ein Fragment der regulatorischen Regionen des humanen Vimentingens umfasst,
- **dass** sie wenigstens eines der folgenden Proteine exprimieren: β1- und β2-adrenerge Rezeptoren, Entkopplungsprotein (UCP), Glucosetransporter Glutl und Glut4, Lipoproteinlipase (LPL) und
- **dass** sie sich in reife humane Adipozyten umwandeln lassen, die Fett produzieren, zahlreiche Mitochondrien enthalten und ferner α2_{A}- und β3-adrenergen Rezeptoren sowie das Expressionsprodukt des Ob-Gens exprimieren, und die verglichen mit den immortalisierten humanen Präadipozyten ein umgekehrtes Verhältnis Glut4/Glutl aufweisen.

2. Zellinien nach Anspruch 1, **dadurch gekennzeichnet, dass** sie aus braunem Fettgewebe stammen.

3. Zellinien nach Anspruch 1 oder Anspruch 2, **dadurch gekennzeichnet, dass** das immortalisierende Fragment aus einem Fragment des SV40-T-Onkogens besteht, das für das T-Antigen des SV40-Virus codiert.

4. Zellinien nach irgendeinem der Ansprüche 1 bis 3, **dadurch gekennzeichnet, dass** der Promotor ausgewählt ist aus dem Promotor des SV40-T-Onkogens und dem Vimentinpromotor, und insbesondere dem Nukleinsäurefragment des Vimentinpromotors zwischen den Basen -878 und +93 der regulatorischen Sequenz von Vimentin.

5. Zellinien nach Anspruch 4, **dadurch gekennzeichnet, dass** das Nukleinsäurefragment des Vimentinpromotors die Basen -830 bis -539 und/oder das Fragment -540 bis -140 umfasst, die stromaufwärts der CAP-Site liegen.

6. Zellinien nach Anspruch 4 oder Anspruch 5, **dadurch gekennzeichnet, dass** sie am 7. Februar 1995 unter der Nr. I-1531 bei der Collection Nationale de Culture de Microorganismes (CNCM) des INSTITUT PASTEUR hinterlegt wurden.

7. Zellinien nach irgendeinem der Ansprüche 2 bis 6, welche aus braunem Fettgewebe stammen, **dadurch gekennzeichnet:**
- **dass** sie wenigstens die folgenden Proteine exprimieren: β1- und β2-adrenerge Rezeptoren, Entkopplungsprotein, Glut1 und Glut4, Lipoproteinlipase (LPL);
- **dass** sie sich in reife Adipozyten umwandeln lassen:
. die multilobäres Fett produzieren,
. die zahlreiche Mitochondrien enthalten,
. die die α2_{A}- und β3-adrenergen Rezeptoren sowie das Expressionsprodukt des Ob-Gens exprimieren, und
. die im Vergleich mit den immortalisierten Präadipozyten ein umgekehrtes Verhältnis von Glut4/Glut1 aufweisen.

8. Verfahren zum Erhalt immortalisierter Präadipozyten, welche aus humanem (braunen oder weißen) Fettgewebe stammen, **dadurch gekennzeichnet, dass** es die Transformation von Präadipozyten, die aus humanem (braunen oder weißen) Fettgewebe stammen, mit einer Nukleinsäuresequenz umfasst, die wenigstens ein immortalisierendes Fragment eines viralen Onkogens und wenigstens einen Promotor umfasst, der ausgewählt ist aus der Gruppe, die den Promotor dieses viralen Onkogens und ein Fragment der regulatorischen Regionen des humanen Vimentingens umfasst, wie definiert in irgendeinem der Ansprüche 1 bis 7.

9. Verfahren zur Umwandlung von Präadipozyten nach irgendeinem der Ansprüche 1 bis 7 in reife Adipozyten, wobei das Verfahren **dadurch gekennzeichnet ist, dass** es das Kultivieren der immortalisierten Präadipozyten nach irgendeinem der Ansprüche 1 bis 7 umfasst.

10. Verfahren nach Anspruch 9, **dadurch gekennzeichnet, dass** das Medium ferner wenigstens eine der folgenden Verbindungen umfasst: Glukokortikoide, Triiodthyronin (T3), gegebenenfalls in Verbindung mit Pioglitazon oder seinen Derivaten, β-adrenerge Rezeptoragonisten, NPY und Melatonin.

11. Verfahren zur Selektion und Identifikation von Substanzen, die zur Regulierung von Lipolyse und/oder Thermogenese fähig sind, **dadurch gekennzeichnet, dass** es umfasst:
- In-Kontakt-bringen der immortalisierten Präadipozyten nach irgendeinem der Ansprüche 1 bis 7 mit wenigstens einer Substanz, die fähig ist, sie in Adipozyten zu transformieren,
- In-Kontakt-bringen der erhaltenen reifen Adipozyten mit einer Substanz, die auf wenigstens einen Rezeptor, wie die α2_{A}- und β3-adrenergen Rezeptoren, die Rezeptoren von Insulin und IGF, die ACTH-Rezeptoren und die Rezeptoren des Glucosestoffwechsels (Glut1, Glut4) der reifen Adipozyten, einwirkt, um die Lipolyse und/oder die Thermogenese zu modulieren, und
- Nachweis der Bildung eines Ligand-Rezeptor-Komplexes.

12. Modell zur Untersuchung der von Adipozyten exprimierten Proteine, **dadurch gekennzeichnet, dass** es aus immortalisierten Präadipozyten nach irgendeinem der Ansprüche 1 bis 7 oder aus reifen Adipozyten besteht, die aus diesen immortalisierten Präadipozyten erhalten wurden.

13. Lipolytische Zusammensetzungen, **dadurch gekennzeichnet, dass** sie reife Adipozyten umfasst, die aus immortalisierten Präadipozyten nach irgendeinem der Ansprüche 1 bis 7 erhalten wurden.

14. Modell zur Untersuchung der Regulation der in humanen Adipozyten vorhandenen Gene, **dadurch gekennzeichnet, dass** es aus immortalisierten Präadipozyten nach irgendeinem der Ansprüche 1 bis 7 oder aus reifen Adipozyten besteht, die aus diesen immortalisierten Präadipozyten erhalten wurden.

15. Pharmazeutische Zusammensetzungen zur Bekämpfung von Fettsucht, **dadurch gekennzeichnet, dass** sie reife braune Adipozyten umfassen, die aus immortalisierten Präadipozyten nach irgendeinem der Ansprüche 1 bis 7 erhalten wurden, gegebenenfalls zusammen mit wenigstens einem pharmazeutisch verträglichen Träger.

16. Verfahren zur Untersuchung der Affinität eines von Adipozyten exprimierten Rezeptors für einen oder mehrere bestimmte Liganden, wobei das Verfahren umfasst:
- In-Kontakt-bringen der immortalisierten Präadipozyten nach irgendeinem der Ansprüche 1 bis 7 mit wenigstens einer Substanz, die fähig ist, sie in Adipozyten zu transformieren,
- In-Kontakt-bringen der erhaltenen reifen Adipozyten mit den bestimmten Liganden, und
- Nachweis einer affinen Reaktion zwischen den Adipozyten und den bestimmten Liganden.

17. Kit oder Besteck zum Nachweis einer möglichen Affinität eines Liganden für ein von einem Adipozyten exprimiertes Protein, **dadurch gekennzeichnet, dass** er (es) umfasst:
- eine Kultur von Präadipozyten nach irgendeinem der Ansprüche 1 bis 7,
- Mittel zur Umwandlung dieser Präadipozyten in reife Adipozyten, und
- ein oder mehrere Kontroll-Liganden.
